# DEMANDE DE BREVET EUROPEEN

(11) **EP 2 184 069 A1**
(43) Date de publication de la demande: **12.05.2010**
(21) Numéro de dépôt: 08291049.8
(22) Date de dépôt: 10.11.2008
(51) Int. Cl.: A61K 36/18, A61K 36/28, A61K 36/288, A61K 36/34, A61K 36/534

(54) **Traitement naturel de désintoxication d'alcool, sevrage progressif à l'abstinence totale à long terme.**

(71) Demandeur: Walther, Martine, 78380 Bourgival (FR)
(72) Inventeur: Walther, Martine, 78380 Bourgival (FR)

(57) **Abrégé**

Traitement naturel pour contribuer à prévenir les effets secondaires de l'injection de boissons alcoolisées par voie buccale et pour prévenir ses dérives d'aggravation des effets secondaires.
Possibilité d'un sevrage progressif et d'acquérir une maîtrise pour faire sa propre cure de désintoxication à domicile sans passer par une hospitalisation, en 18 jours en respectant les consignes accompagnées du développement du programme décrit pour aboutir à une abstinence totale d'alcool et préventive.
L'invention concerne:
Composition contenant un mélange d'extraits de cinq plantes:
- 1 - Camomille Allemande
*Matricaria chamomilla ou Matricaria discoïdea*
- 2 - Mélisse
*Melissa oficinalis*
- 3 - Prêle
*Equisetum arvense, Equisetacée*
- 4 - Chardon Marie
*Silybum marianum (ou Carduus marianus)*
- 5 - Echinacée
*Echinacea purpurea.*
Et des excipients acceptables pour l'alimentation destinée à être utilisée comme complément alimentaire ou alicament.

## Description

### Description de l'invention :

- Traitement naturel pour contribuer à prévenir les effets secondaires de l'injection de boissons alcoolisées par voie buccale et ses dérives d'aggravation de ses effets secondaires. Avec la possibilité d'un sevrage progressif pour maîtriser aussi bien son alcoolisme mais aussi et surtout pour avoir une opportunité, par choix, de faire sa propre cure de désintoxication à domicile sans passer par l'hospitalisation, en 18 jours en respectant bien les consignes accompagnées du développement du programme décrit avec l'aboutissement d'une abstinence totale d'alcool et définitive.
   La spéciale formule sera vendue sous forme d'un produit, complément alimentaire naturel diversifié décrit à la fin de la présentation, proposée à la fabrication à différents laboratoires.
- Je désire protéger cette formule, et l'invention de ce produit pour : « Traitement naturel pour cure à domicile de désintoxication d'alcool, sevrage progressif, maîtrise de la consommation d'alcool, jusqu'à l'abstinence totale avec soin et entretien à long terme pour retrouver son optimum capital santé sans rechute ; longévité et prévention des cancers dû aux méfaits de l'alcool et maladies cardiovasculaires et prédiabétiques, de l'hépatite C et la cirrhose du foie ».

**En voici la description :**

### 1°) Il y a tout d'abord l'association de sept plantes :

### -1- Camomille Allemande

### Matricaria chamomilla ou Matricaria discoïdea

### Ses vertus :

- contre les engorgements du foie et état dépressif,
- les maux d'estomac et d'intestine, rein,
- migraine pour ceux qui ne supporte pas l'aspirine ou médicaments chimique contre la douleur,
- la fièvre,
- elle est très efficace contre les troubles digestifs, la tension nerveuse et les allergies,
- elle contient un peu de méllatoline naturelle qui aide à retrouver un sommeil équilibré,
- elle est bonne contre les insomnies des alcooliques chroniques, calme, apaise l'anxiété,
- le mal de dos (souvent par intoxication), et les rhumatismes,
- apaisent les irritations des muqueuses gastriques (souvent dû à l'acide d'alcool),
- est anti-inflammatoire, inflammations du tube digestif, gastrites, spasmes, etc.,
- contient un puissant antispasmodique efficace contre les douleurs musculaires et permet de réduire l'irritabilité et l'agitation nerveuse tout en favorisant le sommeil,
- efficace en cas de rhume, de rhinite allergique ou d'asthme, (l'alcool conduit souvent à des problèmes respiratoires graves : 6000 morts par an),
- état grippal,
- calme les démangeaisons.

### -2- Melisse

### Melissa officinalis

### Ses vertus :

- La mélisse est traditionnellement utilisée pour améliorer les digestions difficiles et limiter flatulences et spasmes intestinaux,
- antispasmodique, digestive et calmante,
- elle est également sudorifique, vermifuge et carminative,
- elle est efficace pour les migraines dues à de mauvaises digestions agissant sur l'humeur, en effet l'alcool met entre 12 h et 24 h à être digéré comparé à un fruit 1 heure et un repas normal 6 heures,
- contre les névralgie (de la tête, de l'oreille, faciales, dentaires),
- l'émotivité, l'anxiété, crises nerveuses, convulsions, épilepsie,
- syncopes, vertiges, bourdonnements d'oreilles,
- spasmes (asthme, digestif, cardiaque)
- déficience intellectuelle (mémoire, mélancolie)
- indigestions,
- anémie (un alcoolique chronique s'anémie petit à petit pouvant aller vers une anémie sévère,
- par une digestion difficile, le corps a davantage soif, et un alcoolique va boire selon sa dépendance en boisson alcoolisée qui le déshydrate d'autant plus qu'il urine davantage, vers une déshydratation avec risque cardiovasculaire,
- a l'avantage d'avoir un effet calmant et apaisant sans être trop hypnotique avec une trop grande envie de dormir, mais suffisamment légère pour favoriser un apaisement vers un bon sommeil de qualité et associé à la Camomille Allemande
- calme les crampes d'estomac, spasmes digestifs, et favorise la sécrétion biliaire, contribuant à dégorger le foie,
- aide à traiter l'apoplexie, l'épilepsie, la léthargie, la mélancolie, la manie,
- La mélisse a une action sédative et soigne les troubles qui ont une origine nerveuse : spasmes intestinaux et digestifs, colites, crampes d'estomac, émotivité, anxiété, palpitations et insomnie,
- elle améliore également les états dépressifs,
- action calmante sur le système digestif complétée par un effet anti-inflammatoire et spasmolytique.

### -3- Prêle

### Equisetum arvense, Equisetacée

### Ses vertus :

- propriété diurétique, reminéralisante, hémopoïétique (provoque la formation de globules rouges), antidégénérative et cicatrisante, une teneur exceptionnelle **en silicium,**
- certaines infections rénales,
- goutte,
- albuminurie,
- hémorragie,
- déminéralisation, tétanie, rachitisme, asthénie, fracture, lésions osseuses diverses (d'autant plus qu'un alcoolique chronique se décalcifie et se déminéralise),
- décalcification,
- oedèmes post-traumatiques, les infections des voies urinaires et rénales, ainsi que la guérison de plaies,
- hypertension (l'alcoolisme chronique fini souvent par faire de l'hypertension),
- athérosclérose (prévention cardiovasculaire)
- arthrose (l'alcoolisme dûe aux toxiques de l'alcool accentue et prédispose à l'arthrose),
- diabète (l'alcoolisme chronique prédispose aux diabètes par l'alcool transformé en sucre rapide)
- nervosisme, elle contient naturellement de la silice qui contribue à fabriquer notre calcium qui a un effet calmant et bonne pour le coeur,
- état cancéreux,
- les infections des voies urinaires, les calculs rénaux,
- la plante séchée donne près de 14 % de cendre dont environ 6,40 de Silice (proportion considérable qui fait de la prêle, la plante la plus riche en cet élément majeur, l'un des douze principaux de notre organisme) (la silice est employée pour l'aide à la désintoxication en alcoologie),
- contient 5,50 de carbonate de calcium et des petites proportions de sulfate de potassium, chlorure de potassium, magnésie, phosphate de calcium, fer, manganèse et en plante fraîche 200 à 260 mg de vitamine C par kg.,
- acide linoléique, oléique, stéarique, linolénique, l'équisétine,
- fer, manganèse, magnésium, sodium et souffre,
- Le potassium lui confère de plus des vertus diurétiques,
- favorise l'assimilation du calcium, aliment essentiel du système nerveux et participe ainsi à son bon fonctionnement,
- favorise la souplesse et la mobilité des articulations,
- Reminéralisation osseuse, douleurs articulaires, consolidation des fractures, prévient la perte osseuse,
- tendinites.

### -4- Chardon Marie

### Silybum marianum (ou Carduus marianus)

### Ses vertus :

- propriété de régénérer les cellules du foie (prévention contre le cancer du foie) dont l'alcoolisme chronique les détruits particulièrement,
- combat l'insuffisance hépatique,
- prévient la cirrhose du foie,
- est tonicardique et hypertenseur (par action stimulante cardiaque, sur les surrénales, le rein et la vaso-constriction périphérique),
- pour les tendances défaillances cardio-vasculaire (pertubation équilibre vago-sympathique dû à l'abus d'alcool,
- diurétique,
- stimulant gastrique,
- tonique,
- laxatif,
- 3 flavonolignanes : Silibimine, Silychristine, et Silydianine connus collectivement comme Silymarine, un médicament très efficace pour foie,
- La Silymarine est un flavonoïde, un hépatoprotecteur très puissant qui favorise l'écoulement de la vésicule biliaire, qui tonifie foie et vésicule, traite calculs biliaires et inflammations en favorisant la reconstruction du foie ; elle permet ainsi des guérisons rapides de diverses affections liées au foie, cirrhoses, hépatites, et notamment l'hépatite C,
- son efficacité est telle qu'on l'utilise comme antidote à l'empoisonnement par l'amanite phalloïde,
- La Silymarine est aussi un antioxydant très puissant, plus puissant même que la vitamine E. Elle évite la destruction des cellules du foie en neutralisant les radicaux libres et les Leucotrienes (produits par l'enzyme Lipoxygénase dont elle inhibe l'activité),
- elle protège le foie des dommages provoqués par l'excès d'alcool absorbé (en particulier la diminution de glutathion dans le foie); après seulement 1 mois de supplémentation en silymarine, les niveaux hépatiques glutathion ont augmenté de plus de 30%, des études montrent que d'autres résultats très positifs ont été obtenus lors de la prise de silymarine dans les cas de cirrhose alcoolique et non alcoolique, d'hépatite toxique et d'hépatite virale,
- hémostatiques.

### -5- Echinacée

### Echinacea purpurea

### Ses vertus :

- L'Echinacée a la propriété d'être traditionnellement utilisée pour stimuler les défenses naturelles de l'organisme,
- utilisée traditionnellement pour traiter : les infections des voies respiratoires supérieures ;
- contient des acides gras, du rutoside, des esters de l'acide caféique (comme l'échinacoside), des polysaccharides aux propriétés stimulantes,
- contre les maux de tête,
- infection des voies urinaires,
- antivirale,
- anti-inflammatoires, antiallergiques, antibiotiques et cicatrisantes,
- favorise la sudation,
- augmente la résistance aux infections.

### -6- Ortie

### Urtica dioica, Linné ; Urticacées

### Ses vertus :

- L'ortie est un tonique général, stimulant le métabolisme et revitalisante.
- C'est une plante nutritive contenant de la silice (facilement extraite dans l'eau), du fer (Fe), du zinc (Zn), du manganèse (Mn), du sélénium (Se), des proteines. Des vitamines : C, béta-carotène pro-A, acide folique B9, acide pantothénique B5, K. Des minéraux : potassium (K) , calcium (Ca), magnésium (Mg), phosphore (P).
- En cas de stress, l'ortie inhiberait les effets de l'adrénaline.
- Anti-allergique, Antiasthmatique, glycémiorégulateur, galactagogue, antirhumatismal et diurétique.

### -7- Pissenlit

### Taraxacum officinale

### Ses vertus :

- Diurétique, dépurative et tonique, amie du foie.
- Riche en bêta carotène et en vitamine C dont leurs effets antioxydants nous protègent contre le cancer.
- Contient des fibres, minéraux, du fer et du calcium à poids égal, plus riche que le lait.
- Bonne pour la goutte, l'obésité, l'hypertension, l'artériosclérose, les calculs rénaux.

### Et des excipients acceptables pour l'alimentation destinée à être utilisée comme complément alimentaire ou alicament.

### - Et on peut en rajouter une si :

### -8- (pour ceux qui y ont droit en demandant à son médecin) MILLEPERTUIS (HYPERICUMPERFORATUM)

**Ses vertus :** **antidépresseur** : (pratiquement autant que le Zotoft, à peu de chose près, mais sans les effets secondaires)
- astringente,
- antibactérien (contre les gram +, staphylocoque doré),
- antiseptique,- sédatif, photosensibilisant
- l'anxiété, agit sur la bonne humeur sans somnolence ou trouble de concentration.

### 2°) L'excipient :

### -1- L'huile de Noix

### première pression à froid

### Ses vertus :

- contient de l'oméga 3 qui aide à combattre les dépressions nerveuses en comblant une carence, protectrice et prévention cardiovasculaire et cérébrale.

### -2- L'huile de Germe de blé

### première pression à froid

### Ses vertus :

- contient de la vitamine E contribuant dans le traitement de l'aide au sevrage et de la prévention de l'alcoolisme. Conservateur.

### -3- Gelée royale d'abaille dans du miel

### sucre toléré pour les diabétiques

### Ses vertus :

- aide au sevrage du sucre rapide,
- contient des oligo-éléments, acides aminés, vitamines...
- (un alcoolique chronique se déminéralise et se dévitalise ainsi que s'anémie au fil du temps et a dû mal à s'alimenter n'ayant plus trop d'appétit de ce fait, re-stimuler la faim et permet d'apporter les vitamines essentielles à la vie et un petit apport de protéines et des minéraux.

### -4- Acérola (Malpighia punicifolia)

### vitamine C naturelle dans une petite cerise tropicale

### Ses vertus :

- défenses naturelles, la vitamine C contribue à aider l'organisme à détoxiquer des métaux lourds et redonne forme et vitalité,
- anti-fatigue et fixe le fer,
- contient aussi des vitamines A et B, du Calcium, du Phosphore,
- 4,5g de fruit d'Acérola contient autant de vitamine C que dans un kilo de citron ! C'est à dire 100 fois plus que dans une orange,
- pour les personnes mangeant peu de fruits et légumes. Les fumeurs sont aussi concernés car une simple cigarette détruit l'équivalent d'un verre de jus d'orange en vitamine C,
- puissant antioxydant et antiveillissement,
- anti-infectueuse, renforce le système immunitaire, contre les microbes et les virus en activant la synthèse des anticorps,
- contribue à la formation du collagène des os, des cartilages, des dents, de la peau, des vaisseaux sanguins,
- d'épuisement nerveux, d'asthénie, d'anorexie, de cachexie,
- antistress à prendre sous forme de cure, en cas de surmenage, dépression ou angoisse,
- pouvoir détoxicant.

### -5- Eau (eau de source gazeuse)

### sans nitrate

### Avec des vertus :

- qui pourrait contenir du magnésium, calcium, silice.

### 3°) Trois compléments alimentaires :

### -1- Spiruline

### Micro algue presque aussi vieille que la vie sur terre appelée aussi "cyanobactérie Arthrospira Platensis"

### Ses vertus :

- C'est d'abord l'aliment le plus riche actuellement connu en protéines (60 à 70% en poids sec) et ces protéines sont d'excellente qualité puisqu'elles contiennent tous les acides aminés essentiels,
- On peut la conserver quelques jours au réfrigérateur ou en lui ajoutant 5 à 10% de sel de cuisine et en la recouvrant d'huile,
- Mais en général pour sa conservation elle est séchée, réduite en granulés fins ou en poudre et conditionnée à l'abri de l'air et de la lumière,
- **très stimulante** surtout pour des efforts physiques ou intellectuels à fournir (cas des athlètes, des étudiants, des malades ...),
- contient de l'acide gamma-linolénique, une substance qui fait partie de la famille des acides gras oméga,
- traiter la leucoplasie (inflammation précancéreuse des muqueuses de la bouche) et prévenir sa transformation en cancer;
- **stimule le système immunitaire,**
- contribue à faire baisser le taux de cholestérol,
- elle a des **propriétés détoxicantes,**
- source exceptionnelle de **caroténoïdes** variés (bêta-carotène principalement, mais aussi cryptoxanthine, lutéine, zéaxanthine, etc.), soit autour de 22 mg/5 g. Elle fournit notamment une quantité astronomique de bêta-carotène, soit de 12 000 UI à 25 000 UI par 5 g,
- Elle constitue une excellente source de fer, soit de 3 mg à 8 mg par 5 g,
- en naturopathie, on l'emploie comme complément pour le traitement de l'hépatite C.

### -2- La Levure de Bière

### Contient les Vitamines du groupe B

### Ses vertus :

- **Vitamine B1 *(thiamine) :***
   Ses fonctions : fournie l'énergie au fonctionnement du corps et protège le système nerveux et la transmission de l'influx nerveux. Favoris l'utilisation des sucres.
   Sa carence entraîne par un trouble de son absorption une maladie grave inflammatoire du système nerveux et du coeur le béribéri.
- **Vitamine B2** ***(riboflavine)** :*
   Ses fonctions : beauté de la peau, des ongles, des cheveux et renforce la vision, elle aide à la transformation des sucres, des graisses et des protéines.
      Sa carence est normalement rare.
**- Vitamine B3 ou PP *(acide nicotinique ou niacine) :***
   Ses fonctions : favorise la circulation sanguine et la digestion, participe à la synthèse des acides gras et de certaines hormones.
   Sa carence peut donner une inflammation des muqueuses digestives, des troubles nerveux et mentaux et une alopécie.
**- Vitamine B5 *(acide pantothénique) :***
   Ses fonctions : antistress et favorable excellente pour l'équilibre nerveux et elle est indispensable pour l'assimilation des nutriments. Intervient également dans la synthèse du cholestérol.
      Sa carence : entraîne fatigue et des troubles du sommeil et de la coordination.
- **Vitamine B6** ***(pyrodoxine)*** :
   Ses fonctions : antifatigue, stimule les défenses immunitaire et est antidéprime.
   Bénéfique pour la peau et les muqueuses.
   Sa carence : conduit à de l'irritabilité, des convulsions, des troubles digestifs, baisse des anticorps.
- **Vitamine B8 ou H** ***(biotine)** :*
   Ses fonctions : contrôle du poids, antifatigue, santé de la peau, des muscles et des phanères.
   Sa carence : nausées, somnolence, douleurs musculaires, dermatite.
- **Vitamine B9** ***(acide folique)** :*
   Ses fonctions : synthèse de l'hémoglobine. Evite la fatigue et l'irritation, favorise le sommeil et entretien la mémoire.
   Sa carence : conduit à une anémie, des troubles gastro-intestinaux, perte de mémoire et pendant la grossesse une malformation congénitale.
- **Vitamine B12 *(cobalamine) :***
   Ses fonctions : elle permet la synthèse de l'ADN. Indispensable à la production et à la maturation des globules rouges ainsi qu'à leur multiplication.
   Elle optimise aussi les fonctions du cerveau et du corps.
   Sa carence : provoque une anémie pernicieuse (en raison de son évolution fatale) et des troubles neurologiques.

### -3- Le Pollen de fleurs

***Un petit apport complet équilibré de vitamines et minéraux en restimulant et rééduquant l'appétit***
**Ses vertus :**
Ses fonctions : fournie la vitalité et des vitamines essentielles à la vie que le corps ne peut pas fabriquer et est indispensable à la vie.

### Indications générales :

**asthénies** ou états de fatigue, à tous les degrés, portant aussi bien sur la sphère physique, psychique, sexuelle ou intellectuelle:
- asthénie en cours de maladie
- asthénie post-opératoire
- convalescence
- surmenage
- épuisement physique ou intellectuel
- asthénie du vieillard
- asthénie névrotique ou neurasthénie.

**état de maigreur** sous toutes leurs formes, et tout particulièrement *(chez les enfants)* ou alcoolique souffrant de malnutrition où l'effet est spectaculaire.
- et état carentiels divers, tel le rachitisme, retard de croissance, l'insuffisance pondérale, une mauvaise dentition, etc.

### terrains déficients constitutionnels

**sénescence** ou vieillissement prématuré et anormalement exagéré qui représente une excellente indication.

### Indications particulière

### sphère cardio-vasculaire et sanguine :

- **artériosclérose** et ses conséquences dont **l'hypertension artérielle** qui est souvent favorablement influencée ;
- **fragilité vasculaire** en général et capillaire en particulier ;
- **anémies** simples.

### sphère digestive

- **anorexie** par stimulation de la fonction gastrique ;
- **constipation** sous toutes ses formes ;
- **diarrhées chroniques**, des troubles instestinaux, régulateur de la fonction intestinale ;
- **infections intestinales chroniques** souvent génératrices de colibacillose ;
- **alcoolisme chronique** par action sur l'anorexie et l'amaigrissement.

### sphère génito-urinaire :

- **prostatisme** avec son cortège de troubles désagréables et de complications. C'est ainsi que dans l'adénome de la prostate au stade pré-chirurgical, le pollen donne des résultats très intéressants et qui méritent attention, avec des effets à la fois décongestifs et antiphlogistiques non négligeables *(observations médicales portant sur de nombreux malades et sur plusieurs années) ;*
- **asthénie sexuelle** et certaines formes d'impuissance ;
- colibacillose, pour sa relation fréquente avec les infections intestinales sur lesquelles le pollen est actif.

### sphère neuro-psychique

- neurasthénie (état dans lequel la fatigue névrotique est prédomiinante) ;
- états dépressifs réactionnels à un mauvais état physique ;
- nervosisme et certaines insomnies réactionnelles qui sont souvent régularisées ;
- troubles de la mémoire.

### sphère ostéo-articulaire :

- arthrose ou rhumatisme chronique dont les symptômes désagréables sont parfois soulagés.

### sphère dermatologique :

- fragilité cutanée, et cicatrisations difficiles ;
- ongles fragiles ou cassants, et la chute des cheveux qui est retardée, souvent même stoppée ;
- certaines maladies de la peau en tirent aussi bénéfice.

### sphère visuelle :

- fatigue oculaire,
- troubles de la vision crépusculaire.

### sphère métabolique :

- états de maigreur et de dénutrition sans étiologie précise,
- certains états.diabétiques seraient favorablement influencés par le pollen mais l'expérience est encore insuffisance actuellement.

### vitamines :

- B1, B2, B3 ou vitamine PP, B5 ou acide pantothénique, B6 ou pyridoxine, B7 ou mésoinositol, B8 ou vitamine H ou biotine, B9, ou acide folique, B12 ou cyanocobalamine, C ou acide ascorbique, D, E ou tocophérol, provitamine A ou carotène qui se transforme dans l'organisme en vitamine A).

### oligo-éléments :

- calcium, chlore, cuivre, fer, magnésium, manganèse, phosphore, potassium, silicium, soufre.

### enzymes ou ferments :

- l'amylase, l'invertase, certaines phosphatases. D'autres constituants telle la rutine, substances accélératrice de la croissance.

### substances antibiotiques actives :

- sur toutes les souches de Colibacilles et certaines de Proteus et Salmonelles.

### acides aminés :

- acide aspartique, acide glutamique, alanine, arginine, cystine, glycine ou glycocolle, histidine, isoleucine, leucine, lysine, méthionine, phénylalanine, proline, sérine, thréonine, tryptophane, tyrosine, valine).
*Cette liste contient tous les acides aminés indispensables à la vie (au nombre de 8 : isoleucine - leucine - lysine - méthionine - phénylalanine - thréonine - tryptophane - valine). L'intérêt majeur du pollen.*

### Aloès (Aloe vera)

### Digestive et nutritive, stomachique, cholagogue, laxative.

### Ses vertus :

*Ses fonctions : Des dérivés anthracéniques (spécifiques de la sève) - notamment la barbaloïne (ou aloïne) pour ses propriétés digestives. Contient presque tous les acides aminé. Des éléments minéraux (certains sous forme d'oligo-éléments) : calcium, chlore, cuivre, chrome, fer, lithium, magnésium, manganèse, phosphore, potassium, sodium, zinc. Des vitamines: A - B1 - B2 - B3 ou vitamine PP - B6 - B9 - B12 - C - E. Des enzymes: amylase, catalase, cellulase, lipase, oxydase et phosphatases.*

### 4°) OLIGO ELEMENTS :

### -1- ZINC (de 2 ml)

### Renforce les défenses immunitaires

### Ses vertus :

- Antioxydant, renforce l'immunité cellulaire profondément abaissée par le stress, par la grande sensibilité des lymphocytes à ces radicaux.
   Lutte contre un excès de radicaux libre.
   On retrouve une baisse de zinc, teneur célébrale, dans la maladie d'Alzeimer. Surchargé en aluminium.
   Rôle important dans le métabolisme des protéines, des glucides et des lipides.
   La carence en zinc peut entraîner des problèmes de cicatrisation, trouble du goût et des anomalies de la maturation sexuelle.
   Il interviendrait dans la prévention des effets toxiques dus aux radicaux libres. Hypoglycémiant, diabète, complément de traitement.

### -2- SELENIUM (de 2 ml)

### Prévention cancer colorectal et du poumon, antioxidant

### Ses vertus :

- Prévient le stress oxydatif (toxines internes, radicaux libres en excès).
- Renforce les défenses immunitaires, antiradicalaire et aide à prévenir certains cancers. Il est aussi utilisé chez l'adulte comme modificateur du terrain en particulier au cours d'affections musculaires et cutanées.
- Pour les fumeurs, exposition à la pollution, stress, infections, inflammations.
- Indispensable à la fabrication de nombreuses enzymes.
- certaines hypothyroïdies sont aggravées par des carences en sélénium.
- Ce minéral entre également dans la composition de protéines spécifiques des spermatozoïdes, de la prostate...
- Le sélénium est également indispensable pour le cerveau. Car le siège de la pensée doit éliminer les radicaux libres, pour garder ses capacités au top.
- Un manque de sélénium est associé à une augmentation des épisodes dépressifs et de divers troubles de l'humeur, tel que l'anxiété.
- Bon pour les yeux grâce à ses propriétés antioxydante, prévient dégénérescence maculaire liée à l'âge. Dans 20 % des cas, elle entraîne une cécité définitive, lié entre autre à l'action de composés nocifs en excès, les fameux radicaux libres leur présence importante dans l'oeil serait en partie expliquée par un manque en sélénium et d'autres composés antioxydants (vitamines C et E) dans le sang.

### -3- MAGNESIUM (de 2 ml)

### Traite la dépression. Aide à l'élimination.

### Ses vertus :

- Aide à l'élimination. Prévient et combat les crampes.
- Traite la dépression qui calme les nerfs et apaise, aide à soigner la spasmophilie en rééquilibrant le calcium-magnésium, (diminuer la consommation de produits laitiers voir complètement en cas de tétatine, spasmophilie), aide à prévenir le cancer, équilibre le système nerveux.
Il est utilisé chez l'adulte comme modificateur du terrain en particulier au cours d'états de dystonie neurovégétative et d'états regroupés sous le terme de spasmophilie.

### -4- LITHIUM (de 2 ml)

### Traite la dépression et l'irritabilité.

### Ses vertus :

- Comble sa carence souvent pour les patients qui ont été longtemps dépressifs.
- Pour les états dépressifs et d'irritabilités au cours de manifestations pyschiques et qui sont souvent déficitaire en Lithium. Aide à soigner la dépression et la maniacocité.
   Comble les carences en cet oligo élément (pas de traitement prolongé pour effet secondaire faiblesse musculaire et ferait l'effet contraire désiré de l'anti-dépression c'est à dire conduirait à rendre un état suicidaire. Il ne faut pas l'associer à d'autres médicaments et il est déconseillé pendant la grossesse et l'allaitement. Les femmes dépressives après la grossesses, c'est la plupart du temps due à une carence en oméga 3 qu'il faut recombler après l'accouchement pour ne pas avoir la déprime classique dans ces mois suivant et durant l'allaitement). Et je dirais la même chose pour les alcooliques chroniques.
- **Ne pas associer avec du Zoloft** (antidépresseur classique) (par exemple ou autre...) interactions médicamenteuses, pourrait entraîner des accidents cardiovasculaires.

### -5- CUIVRE-OR-ARGENT (1 petite cuillère)

### Traite la fatigue et aide à l'adaptation.

**Ses** vertus :
- Anti-inflammatoire, également aide à cicatriser..

### -6- MANGANESE (de 2 ml)

### Modificateur du terrain au cours d'états allergiques.

### Ses vertus :

- La maladie d'alcoolisme chronique est aussi due à une allergie à l'alcool.
- Est utilisé chez l'adulte comme modificateur du terrain en particulier au cours d'états allergiques.
   (Pour les personnes ne mangeant pas beaucoup de fruits et légumes, et déminéralisée par l'alcool, aide à renforcer les défenses immunitaire et à combattre les infections. (A éviter pendant la grossesse et l'allaitement ou demander avis à votre médecin traitant).

Toute cette description, des chapitres développés ci-dessus : N° 1 à 4, pourra être conditionnée sous forme d'ampoule ou de gellule. Pour la fabrication d'un alicament ou d'un complément alimentaire pour mettre en oeuvre pour une prévention de la maladie chronique de l'alcoolisme, pour une cure de désintoxication d'alcool ou un sevrage alcoolique, et/ou maîtriser l'alcoolisme, (peut aider aussi au sevrage, à l'arrêt du tabac et sa convalescence).

Et avec cette utilisation pour maîtriser et aider à se désaccoutumer de l'emprise de l'alcool par éventuelle prise supplémentaire et simultanée d'un mélange ajouté constitué décrit ci-dessous : La description de ce mélange ajouté devra se prendre pour commencer au tout début du traitement pendant les premiers jours de désintoxication dans 2 à 3 livres d'eau par jour (pour ensuite ajouter un peu de charbon activé avant de reprendre la description des chapitres N°1 à 4 et en cas de rechute passagère ou le lendemain de la dite « gueule de bois » (et en reprenant ensuite selon la description des chapitres 1 à 4 pendant 2 à 3 mois ou par cure de 18 jours. Sous la forme d'ampoule ou de gélule ou d'alicament.

La première semaine, on boira sans une goutte d'alcool, ni boisson dite sans alcool, ni aliment ou médicament à base d'alcool, deux à trois litres d'eau argileuse verte (contient du lithium, de l'aluminium, silice, fer) (la valeur d'une petite cuillère sans mettre le contact de l'argile verte avec le métal dans un litre d'eau macéré la veille, et le lendemain, le boire pendant 3 jours), et un comprimé de charbon activé (pour une détoxication lourde) pendant 2 jours et du jus d'orange pur à 5 heures du soir avec des gouttes homéopathiques ci-après :

### - Ajouter, en demandant avis à votre médecin traitant si vous ne Prenez Pas d'autres traitements médicamenteux

### Hypericum perforatum : en 9 CH. 5 à 10 gouttes. En eau distillée.

### (*C'est la plante du Millepertuis)

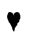 **Silicea :** en 7 CH. *5 à 10 gouttes. En eau distillée.*
   *(*On trouve de la silice naturelle dans la plante de la prêle des champs) Très reminéralisante et détoxicante, aide à l'élimination.*
   **Carduus Marianus :** en 7 CH. 5 *à 10 gouttes. En eau distillée.*
   ***(*C'est la plante du Chardon Marie)***
   *Traditionnellement utilisé dans les troubles fonctionnels digestifs attribués à une origine hépatique. Régénère les cellules du foie.*
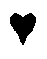 **Echinacea angustif. :** en **7 CH. 5 *à 10 gouttes. En eau distillée.***
   *(*C'est la plante de L'échinacée)*
   *Plante puissante antivirale et renforce tout le système immunitaire.*
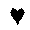 **Phosphorus :** en **7 CH. *5 à 10 gouttes. En eau distillée.***
   *Une étude positive sur les souris pour le traitement de l'hépatite C.*
   *C'est utilisé en homéopathie également pour la prévention de la maladie de l'alzeimer.*
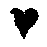 **Sulfur :** en **9 CH. *5 à 10 gouttes. En eau distillée.***
   *A prendre avant de commencer le traitement pour une Première élimination d'attaque.*

### Et seulement après la deuxième semaine on prendra le sirop d'après la composition de la première description citée avant ci-dessus.

Pour la fabrication on procède ce cette façon :
- On prépare la tisane des plantes citée en première description fraîche ou séchée dans de l'eau bouillante de l'eau de source sans nitrate en laissant macérer jusqu'au refroidissement.
- Ensuite on mélange la gelée royale d'abeille, l'huile de noix première pression à froid, la poudre de la cerise Acérola de vitamine C, la levure de bière et la spiruline ainsi que le pollen de fleur.
- On rajoute les oligo-éléments cités.

Selon dans la fabrication pour la diversification pour ceux qui ne supporte pas un produit spécial cité dans le contenu, on retranchera un des produits rajoutés cités.

FIN de l'explication et description de l'invention pour soigner, prévenir et traiter l'alcoolisme chronique. Madame Martine WALTHER

## Revendications

1. **Composition contenant un mélange d'extraits de sept plantes :**
- **1** - **Camomille Allemande**
*Matricaria chamomilla ou Matricaria discoïdea*
- ***2*** - **Mélisse**
*Melissa officinalis*
- ***3*** - **Prêle**
*Equisetum arvense, Equisetacée*
- ***4*** - **Chardon Marie**
*Silybum marianum (ou Carduus marianus)*
- ***5* - Echinacée**
*Echinacea purpurea*
- ***6*** - **Ortie**
*Urtica dioica, Linné ; Urticacées*
- ***7*** - **Pissenlit**
*Taraxacum officinal.*
**Et des excipients acceptables pour l'alimentation destinée à être utilisée comme complément alimentaire ou alicament.**

2. **Composition de complément alimentaire selon la revendication, caractérisée en ce qu'elle peut de plus éventuellement contenir des extraits de :**
- **Millepertuis**
*Hypericum perforatum.*

3. **Composition selon l'une des revendications 1 ou 2, caractérisée en ce que les excipients consistent de :**
- L'huile de noix, et/ou l'huile de germe de blé, et/ou gelée royale d'abeille dans du miel, et/ou Acérola *(Malpighia punicifolia)* et/ou eau *(eau de source gazeuse sans nitrate).*

4. **Composition selon l'une des revendications 1 à 3, caractérisée en ce que, elle peut de plus contenir :**
- de la Spiruline *(Cyanobactérie Arthrospira Platensis)*
- et/ou de la Levure de Bière
- et/ou du Pollen de Fleurs
- et/ou de l'Aloès *(Aloe vera).*

5. **Composition selon l'une des revendications 1 à 4, caractérisée en ce qu'elle peut de plus contenir :**
- Des oligoéléments en ampoule de 2 *ml* tels que du Zinc, du Sélénium, du Magnésium, du Lithium, du Manganèse et du Cuivre-or-argent *(1 petite dose cuillère).*

6. **Composition selon l'une des revendications 1 à 5, caractérisée en ce qu'elle peut être conditionnée sous forme d'ampoule ou de gélule.**

## Revendications modifiées

### Revendications modifiées conformément à la règle 137(2) CBE.

**7.** Utilisation de la composition selon l'une des revendications 1 à 6, pour la fabrication d'un alicament ou d'un complément alimentaire pour mettre en oeuvre pour une prévention de la maladie chronique de l'alcoolisme, pour une cure de désintoxication d'alcool ou un sevrage alcoolique, et/ou maîtriser l'alcoolisme. (Peut aider aussi au sevrage, à l'arrêt du tabac et sa convalescence).

**8.** Utilisation de la composition selon l'une des revendications de 1 à 6 pour préparer un médicament destiné à maîtriser et aider à se désacoutumer de l'emprise de l'alcool par éventuelle prise supplémentaire et simultanée d'un mélange constitué de :
Tout en eau distillée :
- Hypericum perforatum en 9 CH, 5 à 10 gouttes
- Silicea en 7 CH, 5 à 10 gouttes
- Carduus Marianus en 7 CH, 5 à 10 gouttes
- Echinacea angustif en 7 CH, 5 à 10 gouttes
- Phosphorus en 7 CH, 5 à 10 gouttes
- Sulfur en 9 CH, 5 à 10 gouttes,
à prendre pour commencer au tout début du traitement pendant les premiers jours de désintoxication dans 2 à 3 litres d'eau gar jour (pour ensuite ajouter un peu du charbon activé et d'argile verte avant de reprendre la revendication de 1 à 7):
- et en cas ce rechute passagère ou le lendemain de la dite « gueule de bois », (et en reprenant ensuite selon la revendication de 1 à 7pendant 2 à 3 mois ou par cure de 18 jours).
